# EUROPEAN PATENT APPLICATION

(11) **EP 1 106 065 A2**
(43) Date of publication of application: **13.06.2001**
(21) Application number: 00125230.3
(22) Date of filing: 22.11.2000
(51) Int. Cl.: A01N 1/02, C12M 1/26, C12N 5/02

(54) **Method of and container for storing and transporting vital tissue, fluid or cell materials**

(30) Priority: 26.11.1999 DE 19956983; 31.03.2000 DE 10016163
(71) Applicant: CellContol Biomedical Laboratories GmbH, 82152 Martinsried (DE)
(72) Inventor: Metzger, Rainer, Dr., 86899 Landsberg am Lech (DE); Gerhardt, Christina, 32425 Minden/Westfalen (DE)
(74) Representative: VOSSIUS & PARTNER

(57) **Abstract**

A method of storing or transporting living cells of a subject is described, wherein during storage or transport the cells are located in a medium enriched with a fluid occurring naturally in the subject's body from which the cells originate.

A container for transporting vital cell and tissue material as well as body fluids of any kind is also described.

## Description

The present invention relates to a method of storing or transporting living cells and cell compartments of a subject, in particular vital tissue, fluid or cell materials, wherein during storage or transport the cells are located in a medium enriched with a fluid occurring naturally in the subject's body from which the cells originate.

The present invention further relates to a container for storing or transporting vital cell and tissue material as well as body fluids of any kind. Transport containers of that kind are used for taking and stably transporting corresponding biological samples to be taken to an examination, for example in order to determine the effects of test substances. Such transport containers are used in the fields of clinical analysis and diagnosis, in chemo-, immuno- and gene therapy, in pharmacology, such as in the context of clarifying pharmacodynamic mechanisms of action, or for toxicological testing of substances or food-induced allergies.

It is known to ship organ tissues, punch biopsy cells and fine needle aspirates in an adapted transport medium to analytical laboratories to be selectively prepared for the specific examinations and tests there.

In the past, the conditions of shipment (type of packaging, containers, transport media, etc.) and the accompanying deterioration of the sample material have not sufficiently been taken into account. The transport medium frequently has a diverse composition. It contains components which, for example, originate from animals or from human plasma samples and fail to possess individual components adapted to the patient's (subject's) material to be examined. This situation will occur when cells (such as tissue samples, biopsy samples, excision samples) are removed from their natural body environment by artificial isolation so that associated and stabilizing factors for the material to be examined get lost and are no longer made available in the transport medium.

This may lead to inadequate supply of necessary components to the cells and may trigger non-physiological changes in the cells.

If cells in this situation are subjected to subsequent monitoring by diagnostic testing (e.g., an interaction with reference or solid substances), the test result for later analysis may lead to a false diagnosis. This may affect in vitro test systems employed to study the behavior of an organ or the tissue in question or to assess the systemic status of a patient.

Prior transport containers and transport media hardly take these physiological conditions for storing and transporting cells into account, so that there exists an urgent need to improve these containers and media in view of a clearly increasing number of in vitro and ex vivo examinations.

An example of a prior art container is given in EP-A-0 512 769.

It is an object of the present invention to provide a storage or transport method as well as a container for storing or transporting vital tissue, fluid and cell materials in which the sample is stabilized and its vitality and natural functional capability is maintained to the greatest possible extent. Consequently, it is also an object to provide methods as well as a transport container allowing sampled tissues to be transported in an environment close to their origins so that subsequent examinations may produce reliable information about the tissue from which the sample was taken.

This object is solved by the embodiments specified in the accompanying claims.

Thus, the present invention relates to a method of storing or transporting living cells of a subject, wherein during transport the cells are located in a medium enriched with a fluid occurring naturally in the subject's body from which the cells originate.

The use of a subject's body fluids according to the invention for storing or transporting living cells of that same subject guarantees a higher stability of the sample and ensures that the sample is subjected to less influence from outside the body that could falsify the results of examinations to be possibly performed, such as immunoreactions with substances foreign to the body. In addition, storage and transport times may be prolonged significantly without damaging the cells.

The term "subject" is meant to include any type of organism from which living cells can originate. Preferably it specifies a human organism.

Fluids occurring naturally in the body are in particular any fluids an organism of origin is capable of producing, such as blood, tissue fluid, plasma, secretion, sputum, saliva, urine, synovial fluid. The subject's body fluid is preferably serum obtained from the subject's blood.

The subject's serum may be obtained in a conventional manner known to the person skilled in the art, for example by means of separation by centrifuging at 2000 x g for about 15 minutes or by using a suitable filter (available from Sartorius, Gottingen, Germany, or Sarstedt AG, Nurmbrecht, Germany). Methods of separating body fluids into cellular and liquid components are also described in Thomas (Labor und Diagnose, 5^{th} edition, TH-Books Verlagsgesellschaft mbH (1998), 1494 seq.), in Eberhagen (Klinische Chemie und Hämatologie, 3^{rd} edition, Urban & Schwarzenberg Verlag (1973), 8 seq.), and in Jacobs, Wayne, Paul (Laboratory Test Handbook, 2^{nd} edition, Lexi-Comp. Inc. Hudson (1994), 21 seq.).

In a preferred embodiment of the method, a transport container according to the invention as described below is used for transporting the sample, and the subject's body fluid is obtained by using the transport container as explained in detail below.

In general, the subject's body fluid is not used alone for storing and transporting the cells, but is added to a medium suitable for storing cells. Examples of suitable media are RPMI medium or DMEM medium, if necessary including standard additives such as antibiotics (e.g., penicillin, streptomycin, kanamycin, mefan), antimycotics (e.g., amphotericin) and fetal calf serum (FCS). Preferably, the subject's body fluid is added to the medium in an amount yielding a final concentration of the subject's body of for example 10%.

If desired, additional substances or factors may be added to the medium and the subject's body fluid, respectively. Examples include additives preventing bacterial or mycobacterial contamination as well as additives ensuring a high stability of the sample. In particular, these include antibiotics and antimycotics as well as antibiotics and antimycotics mixtures, preferably those specifically adapted to the respective tumor origin and tumor type, or those specifically adapted to nosocomial germs, especially to germs occurring in the clinic where the subject is staying and where the biopsy sample is obtained. These also include combinations of antibiotics and antimycotics mixtures.

Adding such antibiotics and/or antimycotics can prevent for expanding of contaminations during storage and/or transport and in the course of a possible subsequent examination of the cells. This will improve the accuracy of examination results obtained, as the examination is not disturbed by germinating bacteria or fungi leading to possible misinterpretation.

The following additives may also be added to the medium and the subject's body fluid, respectively: for example, coagulation factors, preservatives, radioactive isotopes, viral vectors, gene and/or oligonucleotide sequences, immunomodulating factors such as interleukins and interferons, antibodies, enzymes such as collagenases and Dnases, and/or substances capable of influencing cell functions (e.g., actinomycin).

The cells to be stored or transported may be any living or vital cells, such as cell or tissue associations, cell compartments, body fluids or secretions of any kind and origin. The cells preferably comprise cancer cells. The cancer cells may be derived from any type of cancer. Particular examples of types of cancer and tumors, respectively, are mastocarcinoma, ovarian carcinoma, bronchial carcinoma, colon carcinoma, melanoma, carcinoma of the bladder, gastric carcinoma, head/neck tumors, malignant lymphomas, brain tumors, carcinoma of the cervix uteri, prostate cancer, testicular carcinoma, schwannoma, leukemias, leiomyosarcomas, Non-Hodgkin's lymphoma, Hodgkin's lymphoma, thyroid lymphoma, bone cancer, renal carcinoma, cancer of the pancreas and cancer of the esophagus.

The sample may be taken through different ways of removal, for example through fine needle biopsies (fine needle aspirates), punch biopsies, solid tissue pieces, pleural effusions, ascites samples, irrigation liquids, blood samples, urine samples, sperm samples or as spinal and cerebral fluids, entire organs (e.g., adrenal gland), complete organisms (e.g., nematodes). The material thus obtained may be blended and transported with the subject's body fluids. Thus, body fluids may for example be blended with a pleural sample. A pleural sample comprises body fluids taken from the abdominal cavity or the lung and containing portions of tumor cells. Moreover, body fluids having, for example, serum constituents may be blended with an irrigation solution which is used for scouring tumor cells, for example in the area of the vagina. Equally, body fluids having, for example, serum constituents may be blended with a medium and used with irrigation solutions from the area of the bladder used to scour tumor cells from this area. Body fluids having, for example, serum constituents may also be blended with bone marrow explants such as lumbar fluids. Finally, body fluids having, for example, serum constituents may be blended with a fine needle aspirate, such as one obtained by puncture in case of mastocarcinoma. The various withdrawal techniques are described for example in: Kremer, et al. (Chirurgische Operationslehre, Georg Thieme Verlag, Stuttgart/New York (1999), Volume 4, p. 186; Volume 7, 2, p. 370; Volume E, pages 146 to 147 and Volume 5, p. 238), Pichlmayr and Lohlein (Chirurgische Therapie, Springer Verlag (1991), pp. 225, 130, 89, 93, 116, 546 and 547), Malte (Angiologie in Klinik und Praxis, Georg Thieme Verlag (1998), p. 258), Niethard and Pfeil (Orthopädie, Hippokrates Verlag Stuttgart, p. 38) and Bonk (Biopsie und Operationspräparat; Kompendium fur Arzte und Studenten).

The present invention further relates to the use of a fluid occurring naturally in the body of a subject for storing or transporting cells originating from the same subject. The body fluid may be any kind of fluid an organism of origin is capable of forming (see above).

The invention also relates to a transport container as defined in claims 7 and 14. Preferred embodiments of the transport container according to the present invention are specified in the dependent claims.

According to one embodiment of the invention, the transport container comprises two chambers. According to another embodiment, these chambers are independent from each other.

A first embodiment of the container according to in invention is described in the following.

One of these chambers (chamber A) is used to receive body liquids. This chamber may for example be designed in the form of a tube of various sizes provided with an opening at the end to which e.g. an injection needle, a hollow needle, or an adapter may be attached.

In a preferred embodiment, the side where the opening is located is formed such that in place of the opening for an injection needle an open female thread is provided into which a (commercially available) blood sampling system may be screwed. In this case, the outside is preferably provided with another thread onto which for example a firmly closable jacket tube may be screwed.

The second chamber (chamber B) may receive the vital tissue, fluid and cell material to be transported. The two chambers are separated from each other by a movable plunger (separator element) designed as a filter. This plunger is used for drawing in body fluids, and at the same time it serves as a filter for separating disperse fluids. Thus, the first chamber A may for example receive blood of a patient which is separated afterwards into various components, such as serum and blood clot, using the filter plunger. After removing the plunger rod and filling the chamber B, the separated serum may then mix with the cell material. This can ensure that during further transport the vital sample material is permanently stored and transported in a medium containing components that occur naturally in the subject's body and are subject-specific (physiological milieu). Thus, individually adapted stabilization of the sample or vital tissue will be achieved.

The transport container according to the invention significantly improves and prolongs the entire vitality and functional capability of the tissue sample.

This results in a high reliability of information obtained during subsequent diagnosis of the sample.

Generally, cell conglomerates obtained from different biopsies may be transported. Cell materials may be cell and tissue associations, cell compartments, body fluids and secretions of any kind and origin. Preferably, the cell material is a material containing cancer cells, in particular cancer cells taken from a subject.

The device for drawing in body fluids preferably consists of a plunger designed as a filter, the plunger being connected to a rod extending through the second chamber. Therefore, drawing up the plunger produces a vacuum to draw a fluid, such as a blood sample, into the first chamber, while subsequent pressing down onto the plunger, which is designed as a filter having a specified pore size, separates the blood clot from the remaining constituents. Afterwards, the rod may be separated from the plunger at a predetermined weak or breaking point within the first chamber and may be withdrawn from the transport container. The plunger designed as a filter remains in the system to form the partition wall between the body fluid component, such as between serum/plasma and blood clot, and at the same time between the chamber A and the chamber B. At this time, the expressed serum will already be located in the chamber B and may flow around the sample material to be filled in later.

A second embodiment of the container according to the invention is described in the following.

In the second embodiment, the transport container comprises a chamber having substantially the form of a tube. This chamber of the container of the second embodiment corresponds to chamber B of the container according to the first embodiment, i.e. is the chamber that actually contains and transports the vital tissue, fluid and cell material to be transported, and is thus in the following also referred to as chamber "B". Preferably, tube-like chamber comprises at one end a connecting means to which a separating device, for example a filter is connectable. According to one alternative, the filter is directly connectable with the end portion and its connecting means of chamber B, and thus also functions as a lid closing chamber B but allowing to fill chamber B through said filter. According to a further alternative, the filter is connectable with a separate lid which in turn is connectable to said connecting means at the end portion of chamber B. The connecting means is preferably a thread onto which the filter/lid combination or the separate lid may be screwed.

At the other end of the tube-like chamber there is provided a closing means for a screw- or plug type closure, for example a thread onto which a corresponding lid may be screwed.

A further chamber A (corresponding to chamber A of the first embodiment) is used to receive body liquids. This chamber may for example be designed in the form of a tube of various sizes provided with an opening at the end to which e.g. an injection needle, a hollow needle, or an adapter may be attached. Preferably, chamber A is formed as a microvette (available from Sarstedt AG, Nurmbrecht, Germany) or a common syringe.

In a preferred embodiment, the side of chamber A where the opening is located is formed such that an open female thread is provided adjacent to the opening for an injection needle which a (commercially available) blood sampling system may be screwed. In this case, the outside is preferably provided with another thread onto which for example a firmly closable jacket tube may be screwed.

The second chamber (chamber B) may receive the vital tissue, fluid and cell material to be transported. The second chamber is completely separated from the first chamber. Chamber A is provided a with movable plunger. This plunger is used for drawing in body fluids, and it serves further for pressing the body fluids through said filter for separating disperse fluids. Thus, the chamber A may for example receive blood of a patient which is separated afterwards by means of said filter into various components, such as serum and blood clot, using the plunger. The serum received by means of the filter can be filled in chamber B through an opening. Again, this opening is preferably directly connectable with the filter so that the serum is filled in the chamber B through the filter. After filling the chamber B, the separated serum may then mix with the cell material. This can ensure that during further transport the vital sample material is permanently stored and transported in a medium containing components that occur naturally in the subject's body and are subject-specific (physiological milieu). Thus, individually adapted stabilization of the sample or vital tissue will be achieved.

The transport container according to the invention significantly improves and prolongs the entire vitality and functional capability of the tissue sample.

This results in a high reliability of information obtained during subsequent diagnosis of the sample.

Selecting different pore sizes and membrane characteristics for the filter permits a differential separation, for example into large or small cells and/or particles or molecules of different sizes, and, therefore, a specific separation of the drawn-in fluid into cellular and liquid constituents (such as blood) to be effected. The filter is preferably equipped such that if the drawn-in body fluid is blood, the filter permits a separation, for example into serum or plasma and blood clot, to be performed. Generally speaking, blood consists of plasma and blood corpuscles, with the plasma in turn consisting of serum and fibrinogen. Plasma and serum are biological carriers of important properties, such as immunity. Plasma is the liquid constituent of blood. On the other hand, "blood clot" is the cellular constituent of blood. The term "serum" includes the blood constituent that is no longer capable of clotting, i.e., the residual solution cleared from blood corpuscles and fibrin. In human blood, separation into serum and blood clot may for example be achieved by using a filter having a pore size of about 5 µm or less. Such filters may consist of materials known to the person skilled in the art and may for example be obtained from Sartorius, Göttingen, Germany. As the filter, according to the first embodiment, permanently remains in the first chamber to form a partition wall, the separated fraction, e.g., the blood clot, also remains in the transport container during the entire transport, but is separated from the sample material. In the second embodiment, the filter is preferably integrated in chamber B, e.g., in the lid or the bottom of the container.

The invention will be described below with reference to the accompanying figures.
- Fig. 1: shows a container according to a first embodiment of the present invention;
- Figs. 2A, 2B: show a bottom view and a side view, respectively, of a closure for a container according to the first embodiment of the present invention;
- Figs. 3A-3C: demonstrate the use of the closure according to Figs. 2A, 2B;
- Fig. 4: shows a container according to a second embodiment of the present invention;
- Fig. 5: shows a container according to an alternative design of the second embodiment of the present invention; and
- Fig. 6: shows the assembly of the filter module.

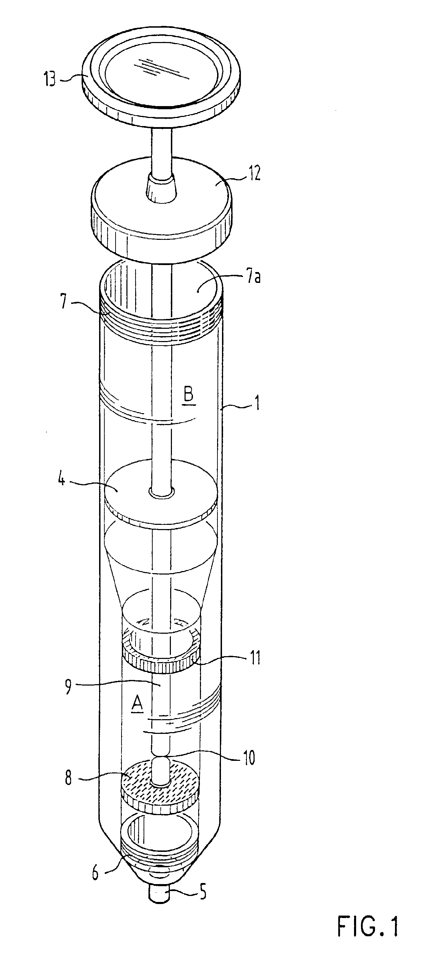

Figure 1 shows a transport container 1 according to a first embodiment which is divided into a first chamber A and a second chamber B. The chamber A is used to receive body fluid such as blood, lymph or the like, which may be taken in using an injection needle or another sampling system. The chamber B serves to store and transport living cell material.

The chamber A has a test tube-shaped construction with an opening 5 to which an injection needle (not shown) may be coupled in the Luer-lock system in order to sample blood or other body fluids, for example. A tube previously filled with a body fluid, such as blood, may be screwed onto a male thread, if provided (see above). A piston 8 is arranged in the chamber A to draw in the fluid by means of a vacuum produced by pulling the piston 8 away from the opening 5 using a piston rod 9 and a handle 13 disposed at the piston rod. The chamber B may include a vacuum-supporting membrane 4 secured to the plunger rod, which is moved together with the plunger by pulling. As a result, the fluid to be sampled, for example blood, flows into the chamber A. On its inside, the chamber A may individually be coated with appropriate substances for stabilizing the respective fluid.

The piston 8 is simultaneously designed as a filter having a pore size suitable for the corresponding application (see above).

After the piston 8 has been withdrawn to a stop 11 forming a locating projection in order to fill the chamber A with the predetermined amount of fluid, the opening 5 is closed by a plug- or screw-type connector 6. Then the piston 8 is pressed back toward the opening 5 so that only fluids or particles of a size limited by the pore size can pass the filter 8 and the constituents not passing the filter are pressed off in the direction of the opening 5.

Thereafter, the piston rod 9 is twisted off at a predetermined breaking point 10 located near the filter 8 and is subsequently removed from the transport container together with the membrane 4. The two chambers are separated by the filter 8. The cell material is filled into the chamber B through the opening 7a and may then interact with the subject-specific components (see below).

The transport container 1 further comprises a closing mechanism 7 on the side of the chamber B to which a screw- or plug-type closure 12 may be applied to close the container on the side of the chamber B as well.

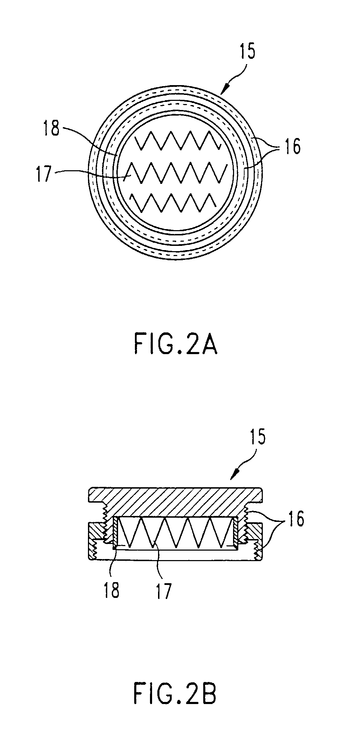

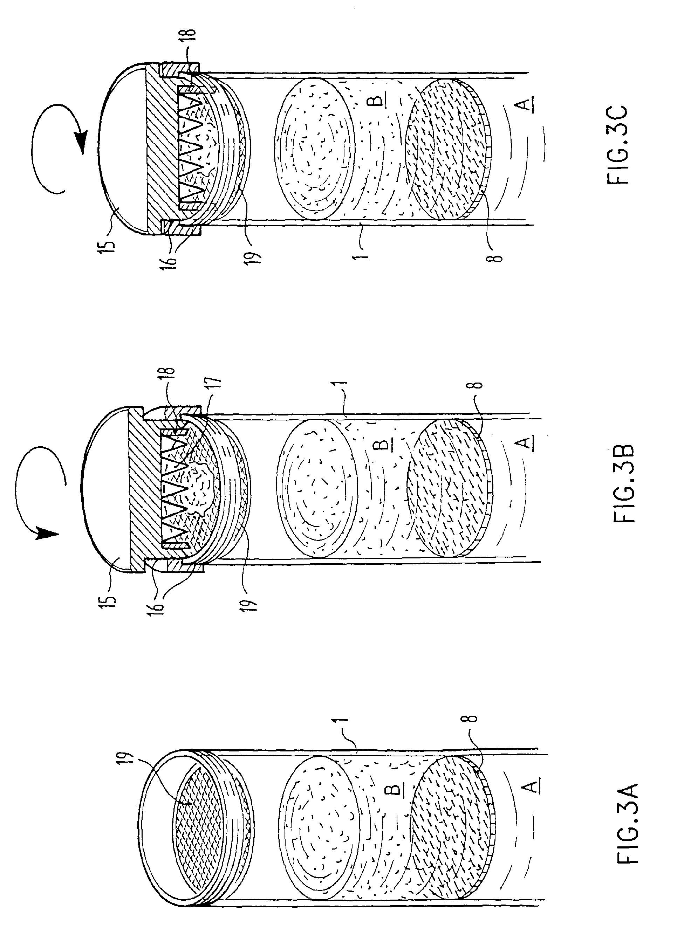

As a preferred embodiment when using the container for transporting larger cell associations such as biopsates or the like, as shown in Figure 2, the chamber B is designed such that the screw-type closure 12 is specifically modified to form a cover 15 having a thread 16 which is first right-handed and then left-handed. The undersurface of the cover is for example provided with knife blades 17 and an annular blade 18 around the edge which is extendable from the cover 15. The blades are used for tissue disintegration and may be employed to comminute pieces of tissue. For this purpose, a sieve 19 is first placed on the chamber B (Figure 3A). The biopsy sample is deposited on top of it. Then, the cover 15 with the bottoming blades is screwed on while turning the thread to the left. As a consequence, the turning motion of the cover macerates the tissue by means of the knife blades 17 (Figure 3B). The back pressure is produced by the sieve 19. In another step, the cover 15 is screwed further into the thread 14 of the chamber B, but this time turning to the right, while a triggering mechanism in the cover is also extending the annular blades (Figure 3C). This causes the sieve 19 including the tissue sample to become separated from the chamber B and transferred into the transport solution. Thereafter, the chamber B is closed by the fluid-tight screw- or plug-type closure 15.

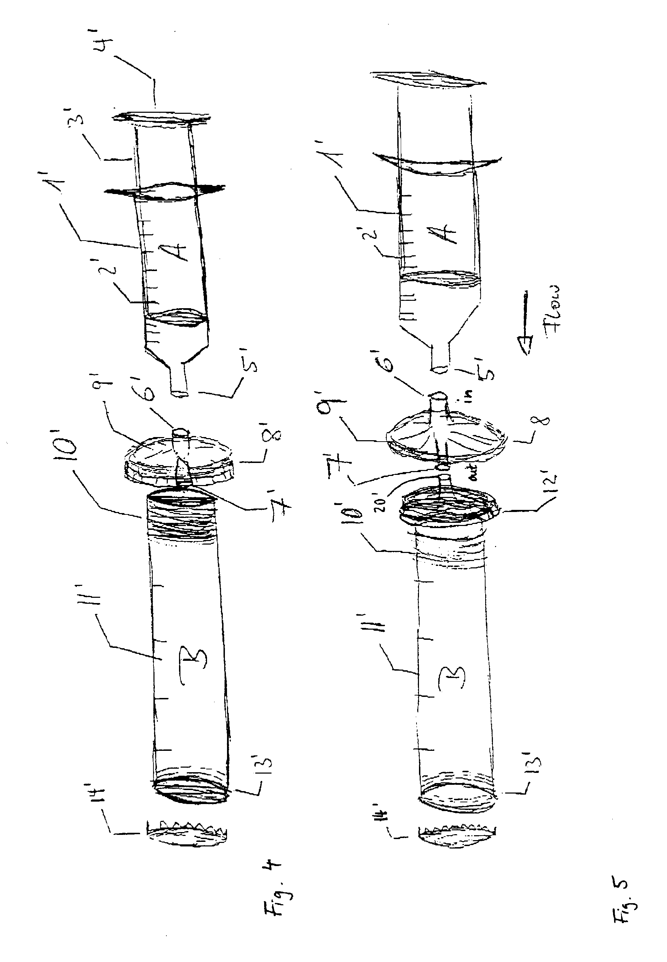

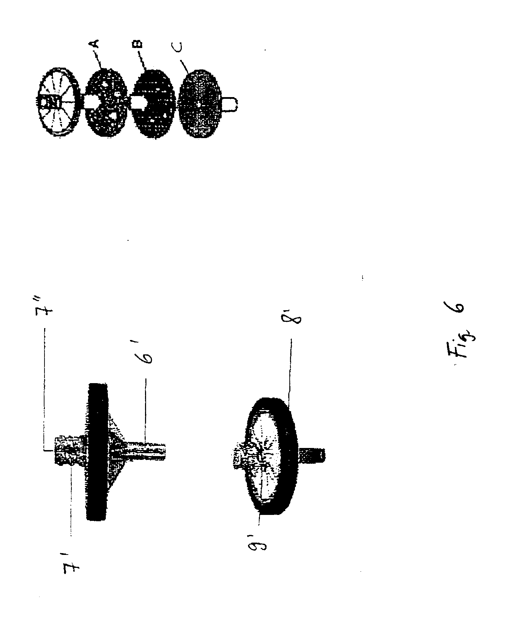

Figures 4 and 5 show a transport container according to a second embodiment which is divided into a chamber A and a chamber B. Both chambers are separate components of the container and independent from each other. The chamber A is used to receive body fluid such as blood, lymph or the like, which may be taken in using an injection needle or another sampling system. The chamber B serves to store and transport living cell material.

The chamber A has a test tube-shaped construction with an opening 5' to which an injection needle (not shown) may be coupled in the Luer-lock system in order to sample blood or other body fluids, for example. A piston 2' is arranged in the chamber A to draw in the fluid by means of a vacuum produced by pulling the piston 2' away from the opening 5' using a piston rod 3' and a handle 4' disposed at the piston rod. The fluid to be sampled, for example blood, flows through opening 5' into the chamber A. On its inside, the chamber A may additionally individually be coated with appropriate substances for stabilizing the respective fluid.

The chamber B (11') is connectable with a filter 8' at the lid end or the bottom end thereof. The filter is a preferably a circular filter 8' and comprises a connector in form of Luer-cone 6' at one side, and a Luer-thread 7' having an internal cone 7" on the other side. After the piston 2' has been withdrawn in order to fill the chamber A with the predetermined amount of fluid, the opening 5' is connected with the filter 8' by connector 6'. Then the piston 2' is pressed towards the opening 5' so that only fluids or particles of a size limited by the pore size can pass the filter substance 9' and the remaining constituents of the blood clot are kept in the filter. The inner part 9' of the filter (e.g., the filter membranes) are preferably provided with additives in form of lyophilisates (e.g., antibioticlyophilisate, anti-coagulant (heparincitrate), or other additives, nutritive substances, proteins) that is solved due to the passage of the serum through the filter and transferred along with the serum into the chamber B and the medium therein.

The dosage of the serum is controlled with a scaling of chamber A and is conducted such that a blending is caused in accordance with the ratio. For example, a part of 3 ml serum can be added to a medium volume of 27 ml. This results in a ratio of 1:10 or a concentration of the serum of 10 % per part, respectively. The cell material as well as the modified transporting medium (e.g., RPMI, DMEM) are filled through the opening 7' into the chamber B, and can now interact with the serum components specific to the tested person (see below). Typically, the medium is already present in chamber B. In the next step the biopsy sample is added, and finally the serum is added from chamber A via the filter.

According to Fig. 4, the filter 8' simultaneously forms a lid for closing chamber B. Chamber B comprises thread 10' for screwing the lid/filter on the chamber B. In an alternative design as shown in Fig. 5, a separate lid 12' is provided that can be screwed onto thread 10'. The lid 12' comprises an attachment portion 20' that is connectable with the internal cone 7" of the filter 8'.

As shown in Fig. 6, the filter 8' comprises a Luer-thread 7' having an internal cone 7". On the opposite side, the filter comprises a Luer-cone 6'. The inner portion 9' of the filter preferably comprises a pre-filter A, and a membrane filter B.

Preferably, the chamber B further comprises a closing mechanism 13' to which a screw- or plug-type closure 14' may be applied to close the container on the side of the chamber B facing away from chamber A as well.

As a preferred embodiment when using the container for transporting larger cell associations such as biopsates, the chamber B is designed such that the screw-type closure 13' is specifically modified to form a cover 14' having a thread which is first right-handed and then left-handed. The undersurface of the cover is for example provided with knife blades 17 and an annular blade 18 around the edge which is extendable from the cover 15 (see Fig. 2). The blades are used for tissue disintegration and may be employed to comminute pieces of tissue. For this purpose, a sieve 19 is first placed on the chamber B (Figure 3A). The biopsy sample is deposited on top of it. Then, the cover 15 with the bottoming blades is screwed on while turning the thread to the left. As a consequence, the turning motion of the cover macerates the tissue by means of the knife blades 17 (Figure 3B). The back pressure is produced by the sieve 19. In another step, the cover 15 is screwed further into the thread 14 of the chamber B, but this time turning to the right, while a triggering mechanism in the cover is also extending the annular blades (Figure 3C). This causes the sieve 19 including the tissue sample to become separated from the chamber B and transferred into the transport solution. Thereafter, the chamber B is closed by the fluid-tight screw- or plug-type closure 15.

The cell material may be introduced into the chamber B as a cell suspension, or may be added as a piece of tissue to a predefined medium filled in previously. Moreover, an ampule-like fluid container may be inserted which, for example, contains transport medium or the like and may be broken or smashed for purposes of thoroughly mixing with the cell material and the drawn-in and separated fluid.

The tissue may also be prepared in advance by adding enzymes or other substances. To do so and to prevent uncontrolled degradation of the tissue, for example, a limited amount of enzymes or other substances may be placed as a lyophilisate or as a wall coating in the chamber B. This permits particular laboratory processes to be performed in the transport container already during transport. Following transport and upon arrival of the sample in the laboratory, the cell material will already be disintegrated, enzymatically pretreated and therefore processed to the largest possible extent, so that the time required for the subsequent laboratory process will be significantly shortened. In this way, the sample is transported under physiological conditions close to its origin, and is processed and supplied to diagnosis within a short period of time.

Transport may be under chilled conditions or at ambient temperatures.

In the transport container described, the presence of subject-specific components from the subject's body is ensured during the entire transport, the sample material is already disintegrated as well as pretreated and processed in a stabilizing manner by corresponding additives. As a result, the sample is in an ideal condition for subsequent diagnostic action.

The present invention further relates to the use of a transport container according to the invention for storing or transporting samples containing living cells, in particular tissue, fluid or cell materials.

## Claims

**1.** Method of storing or transporting living cells of a subject, wherein during storage or transport the cells are located in a medium enriched with a fluid occurring naturally in the body of the subject from which the cells originate.

**2.** Method as claimed in claim 1, wherein the subject's body fluid may be any kind of fluid an organism of origin is capable of forming.

**3.** Method as claimed in claim 1 or 2, wherein the cells include tumor cells of the subject.

**4.** Method as claimed in any of claims 1 to 3, wherein a container as claimed in any of claims 7 to 27 is used for transport.

**5.** Use of a fluid occurring naturally in the body of a subject for storing or transporting cells originating from the same subject.

**6.** Use as claimed in claim 5, wherein the subject's body fluid may be any kind of fluid an organism of origin is capable of forming.

**7.** Container for storing or transporting vital tissue, cell sample materials and body fluids of any kind, comprising a chamber (B) having at least one opening for receiving living cell material such as tissue, fluid and cell sample material, and a device (8') for separating various components of a body fluid being filled in said chamber (B).

**8.** Container according to claim 7, wherein said separating device is connected with said chamber (B) at an opening thereof.

**9.** Container according to claim 7 or 8, wherein said separating device forms a lid of said chamber (B).

**10.** Container according to claim 7, 8 or 9, wherein said separating device (8') comprises a filter element (9').

**11.** Container according to any of claims 7 to 10, wherein said separating device comprises a first connector (6') for connecting said separating means with another chamber (A), said chamber (A) having an opening (5') for receiving body fluids.

**12.** Container according to claim 11, wherein said body fluid is filled from said chamber (A) in said chamber (B) via said separating device (8').

**13.** Container according to any of claims 7 to 12, wherein said separating device comprises a mixture of antibiotics in lyophilized form.

**14.** Container for storing or transporting vital tissue, cell sample materials and body fluids of any kind comprising a first chamber (A) having an opening for receiving body fluids, a second chamber (B) having an opening for receiving living cell material such as tissue, fluid and cell sample material, a device for drawing body fluids into the first chamber (A), and a device arranged in the first chamber for separating various components of the drawn-in body fluid.

**15.** Container as claimed in claim 14, wherein the device for drawing in body fluids is a movable plunger arranged in the first chamber and having a positive fit with the walls of the first chamber (A).

**16.** Container as claimed in claim 14 or 15, wherein the device for drawing in body fluids includes a rod extending through the second chamber (B).

**17.** Container as claimed in claim 16, wherein the rod includes a predetermined breaking point within the first chamber.

**18.** Container as claimed in any of claims 14 to 17, wherein the device for drawing in is simultaneously formed as a device for separating.

**19.** Container as claimed in any of claims 7 to 18, wherein the device for separating is a filter having a predetermined pore size.

**20.** Container as claimed in any of claims 11 to 19, wherein the opening of the chamber (A) for receiving body fluids is formed as a fitting for an injection needle, or is provided with a female thread into which a standard blood sampling system may be screwed, or is provided with a male thread onto which a jacket tube may be screwed.

**21.** Container as claimed in any of claims 11 to 20, wherein the opening of the chamber (A) for receiving body fluids and/or the opening of the chamber (B) for receiving cell material is provided with a closing mechanism for a screw- or plug-type closure.

**22.** Container as claimed in any of claims 7 to 21, wherein chamber (B) comprises a thread arrangement having one thread direction in a first portion and the opposite thread direction in a second portion.

**23.** Container as claimed in any of claims 7 to 22, further comprising a detachable closure for the opening for receiving cell material, said closure comprising a thread arrangement permitting two portions of opposite thread directions.

**24.** Container as claimed in any of claims 7 to 23, wherein an additional sieve for receiving and storing the received cell material is arranged in chamber (B).

**25.** Container as claimed in claim 23 or 24, wherein at least one blade for cutting the cell material and/or the sieve arranged in the second chamber is disposed at the closure on its side facing the second chamber (B) in the applied state.

**25.** Container as claimed in any of claims 7 to 24, wherein the chamber (B) contains a transport medium.

**26.** Container as claimed in any of claims 7 to 25, wherein the chamber (A) is coated with stabilizing substances on its inside.

**27.** Container as claimed in any of claims 7 to 26, wherein the chamber (B) may be precoated with substances or enzymes such that pretreatment of the sample material takes place.

**28.** Use of a container as claimed in any of claims 7 to 27 for storing and transporting living cells.
